# FASCICULE DE BREVET EUROPEEN

(11) **EP 1 468 674 B1**
(45) Date de publication et mention de la délivrance du brevet: **11.11.2009**
(21) Numéro de dépôt: 04290651.1
(22) Date de dépôt: 10.03.2004
(51) Int. Cl.: A61K 8/81, A61K 8/894, A61Q 17/04

(54) **Composition solaire aqueuse comprenant au moins un polymère d'acide acrylamido 2-méthyl propane sulfonique amphiphile et une silicone hydrosoluble, utilisations**
Wässerige Sonnenschutzzubereitung enthaltend mindestens ein amphiphiles Acrylamidomethylpropylsulfonsäurepolymer und ein wasserlösliches Silikon, deren Verwendungen
Aqueous sunscreen composition containing at least an amphiphilic polymer of acrylamido 2-methyl-propane sulfonic acid and a water soluble silicone, uses thereof

(30) Priorité: 14.04.2003 FR 0304650
(43) Date de publication de la demande: 20.10.2004
(73) Titulaire: L'ORÉAL, 75008 Paris (FR)
(72) Inventeur: Candau, Didier, 91570 Bievres (FR); Boutelet, Karl, 75011 Paris (FR); Seyler, Nathalie, 94700 Maisons-Alfort (FR)
(74) Mandataire: Miszputen, Laurent

(56) Documents cités:
- EP-A- 1 069 142
- WO-A-93/05762
- US-A- 5 338 352

## Description

L'invention concerne une composition photoprotectrice comprenant au moins une phase aqueuse, un système filtrant les radiations UV, caractérisée par le fait qu'elle contient :
(a) au moins un polymère amphiphile d'acide acrylamido 2-méthyl propane sulfonique, partiellement ou totalement neutralisé, réticulé ou non-réticulé comportant au moins une chaîne grasse et
(b) au moins une silicone hydrosoluble comportant au moins un groupement polyoxyalkyléné monovalent terminal ou pendant.

L'invention concerne également l'utilisation de l'association d'au moins un polymère amphiphile d'acide acrylamido 2-méthyl propane sulfonique, partiellement ou totalement neutralisé, réticulé ou non-réticulé comportant au moins une chaîne grasse et d'au moins une silicone hydrosoluble comportant au moins un groupement polyoxyalkyléné monovalent terminal ou pendant, dans une composition cosmétique aqueuse comprenant un système photoprotecteur capable de filtrer le rayonnement UV, pour augmenter le facteur de protection solaire.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

Les rayons UVA et UVB doivent donc être filtrés et il existe actuellement des compositions cosmétiques protectrices de l'épiderme humain renfermant des filtres UVA et UVB.

Ces compositions antisolaires se présentent assez souvent sous la forme d'une émulsion, de type huile-dans-eau (c'est à dire un support cosmétiquement et/ou dermatologiquement acceptable constitué d'une phase continue dispersante aqueuse et d'une phase discontinue dispersée grasse) ou eau-dans-huile (phase aqueuse dispersée dans une phase grasse continue), qui contient, à des concentrations diverses, un ou plusieurs filtres organiques classiques liposolubles et/ou des filtres organiques classiques hydrosolubles capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV. Dans de telles émulsions, les filtres hydrophiles sont présents dans la phase aqueuse et les filtres lipophiles sont présents dans la phase grasse.

On connaît dans la demande WO93/05762 des compositions solaires comprenant l'association d'un copolymère acrylamide/acide acrylamido 2-méthyl propane sulfonique réticulé (SEPIGEL 305) et un organosiloxane pouvant être polyoxyalkyléné dans le but de conférer à la peau un toucher doux sans effet collant.

Or, à la suite d'importantes recherches menées dans le domaine de la photoprotection évoqué ci-dessus, la Demanderesse a maintenant découvert, de façon inattendue et surprenante, qu'en ajoutant, dans un support aqueux contenant de 0,1 % à 30 % en poids par rapport au poids total de la composition un système filtrant les radiations UV, un polymère amphiphile d'acide acrylamido 2-méthyl propane sulfonique, partiellement ou totalement neutralisé, réticulé ou non-réticulé et au moins une silicone hydrosoluble comportant au moins un groupement polyoxyalkyléné monovalent terminal ou pendant, il était possible d'obtenir des compositions antisolaires aqueuses ayant des indices de protection améliorés, en tous cas supérieurs à ceux qui peuvent être obtenus avec un tel système photoprotecteur seul.

Dans la suite de la présente description, on entend par « système filtrant les radiations UV » par un agent filtrant les radiations UV constitué soit d'un composé organique ou minéral unique filtrant les radiations UV soit un mélange de plusieurs composés organiques ou minéraux filtrant les radiations UV, par exemple mélange comprenant un filtre UVA et un filtre UVB.

Cette découverte est à la base de la présente invention.

Ainsi, conformément à l'un des objets de la présente invention, il est maintenant proposé une composition photoprotectrice comprenant au moins une phase aqueuse et au moins de 0,1 % à 30 % en poids par rapport au poids total de la composition d'un système filtrant les radiations UV,caractérisée par le fait qu'elle contient :
(a) au moins un polymère amphiphile d'acide acrylamido 2-méthyl propane sulfonique, (AMPS) comportant au moins une chaîne grasse partiellement ou totalement neutralisé, réticulé ou non-réticulé et
(b) au moins une silicone hydrosoluble comportant au moins un groupement polyoxyalkyléné monovalent terminal ou pendant.

L'invention concerne également l'utilisation de l'association d'au moins un polymère amphiphile d'acide acrylamido 2-méthyl propane sulfonique, partiellement ou totalement neutralisé, réticulé ou non-réticulé et d'au moins une silicone hydrosoluble comportant au moins un groupement polyoxyalkyléné monovalent terminal ou pendant, dans une composition cosmétique aqueuse comprenant de 0,1 % à 30 % en poids par rapport au poids total de la composition d'un système photoprotecteur capable de filtrer le rayonnement UV, pour augmenter le facteur de protection solaire.

D'autres caractéristiques, aspects et avantages de l'invention apparaîtront à la lecture de la description détaillée qui va suivre.

Les polymères d'AMPS conformes à l'invention sont amphiphiles à savoir qu'ils comportent à la fois une partie hydrophile une partie hydrophobe comportant au moins une chaîne grasse.

On entend par "chaîne grasse", au sens de la présente invention, toute chaîne hydrocarbonée comportant au moins 7 atomes de carbone.

Les polymères d'AMPS utilisés conformément à l'invention sont des homopolymères ou copolymères, réticulés ou non-réticulés comportant au moins le monomère acide acrylamido 2-méthyl propane sulfonique (AMPS), sous forme libre ou bien partiellement ou totalement neutralisée.

De façon préférentielle, les polymères d'AMPS conformes à l'invention peuvent être neutralisés partiellement ou totalement par une base minérale (soude, potasse, ammoniaque) ou une base organique telle que la mono-, di- ou tri-éthanolamine, un aminométhylpropanediol, la N-méthyl-glucamine, les acides aminés basiques comme l'arginine et la lysine, et les mélanges de ces composés. Ils sont généralement neutralisés. On entend dans la présente invention par « neutralisés » des polymères totalement ou pratiquement totalement neutralisés, c'est-à-dire neutralisés à au moins 90%.

Ces polymères d'AMPS selon l'invention peuvent être réticulés ou non-réticulés.

Lorsque les polymères sont réticulés, les agents de réticulation peuvent être choisis parmi les composés à polyinsaturation oléfinique couramment utilisés pour la réticulation des polymères obtenus par polymérisation radicalaire.

On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 à 10 % en mole et plus particulièrement de 0,2 à 2 % en mole par rapport au polymère.

La chaîne grasse présente dans les polymères de l'invention comporte de préférence de 7 à 30 atomes de carbone, plus préférentiellement de 7 à 22 atomes de carbone et encore plus préférentiellement de 7 à 18 atomes et plus particulièrement de 12 à 18 atomes de carbones.

Les polymères amphiphiles conformes à l'invention ont en général un poids moléculaire en poids allant de 50 000 à 10 000 000, plus préférentiellement de 100 000 à 8 000 000 et encore plus préférentiellement de 100 000 à 7 000 000.

Les polymères amphiphiles d'AMPS selon l'invention peuvent être réticulés ou non-réticulés. Les agents de réticulation peuvent être choisis parmi ceux cités ci-dessus. On utilisera plus particulièrement le méthylène-bis-acrylamide, le méthacrylate d'allyle ou le triméthylol propane triacrylate (TMPTA). Le taux de réticulation varie de préférence de 0,01 à 10% en moles et plus particulièrement de 0,2 à 2% en moles par rapport au polymère.

Les polymères d'AMPS amphiphiles conformes à l'invention peuvent notamment être choisis parmi les polymères amphiphiles statistiques d'AMPS modifiés par réaction avec une n-monoalkylamine ou une di-n-alkylamine en C₆-C₂₂ tels que ceux décrits dans la demande de brevet WO00/31154 (faisant partie intégrante du contenu de la description). Ces polymères peuvent également contenir d'autres monomères hydrophiles à insaturation éthylénique choisis par exemple parmi l'acide acrylique, l'acide méthacrylique ou leurs dérivés alkyl substitués en β ou leurs esters obtenus avec des mono ou polyalkyleneglycols, l'acrylamide, le méthacrylamide la vinylpyrrolidone , l'acide itaconique ou l'acide maléique ou leurs mélanges.

Les polymères préférentiels de l'invention sont choisis parmi les polymères amphiphiles d'AMPS et d'au moins un monomère à insaturation éthylénique comportant au moins une partie hydrophobe ayant de 7 à 30 atomes de carbone et plus préférentiellement de 7 à 22 atomes de carbone et encore plus préférentiellement de 7 à 18 atomes de carbone et plus particulièrement de 12 à 18 atomes de carbone. Cette partie hydrophobe peut être un radical alkyle linéaire, saturé ou insaturé (par exemple n-octyle, n-décyle, n-hexadécyle, n-dodécyle, oléyle), ramifié (par exemple isostéarique) ou cyclique (par exemple cyclododécane ou adamantane).

Ces mêmes polymères peuvent contenir en plus un ou plusieurs comonomères hydrophiles à insaturation éthylènique comme l'acide acrylique, l'acide méthacrylique ou leurs dérivés alkylsubstitués en β ou leurs esters obtenus avec des mono ou polyalkylèneglycols, l'acrylamide, le méthacrylamide la vinylpyrrolidone, l'acide itaconique ou l'acide maléique.

Ces mêmes polymères peuvent contenir en plus un ou plusieurs co-monomères hydrophobes à insaturation éthylènique, comprenant par exemple :
- un radical fluoré ou alkylfluoré en C₇-C₁₈ (par exemple le groupement de formule

   -CH₂)₂-(CF₂)₉-CF₃)
- un radical cholestéryle ou un radical dérivé de cholestérol (par exemple l'hexanoate de cholestéryle)
- un groupe polycyclique aromatique comme le naphtalène ou le pyrène
- un radical siliconé ou alkylsiliconé ou encore alkylfluorosiliconé.

Ces copolymères sont décrits notamment dans la demande de brevet EP-A-750899, le brevet US-A-5089578 et dans les publications de Yotaro Morishima suivantes :
- « Self-assembling amphiphilic polyelectrolytes and their nanostructures - Chinese Journal of Polymer Science Vol. 18, N°40, (2000), 323-336 »;
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
- « Solution properties of miscelle networks formed by non-ionic moieties covalently bound to an polyelectrolyte : salt effects on rheological behavior - Langmuir, 2000, Vol.16, N°12, 5324-5332 » ;
« Stimuli responsive amphiphilic copolymers of sodium 2-(acrylamido)-2-methylpropanesulfonate and associative macromonomers - Polym. Preprint, Div. Polym. Chem. 1999, 40(2), 220-221 ».

Ils sont également décrits dans les demandes de brevet (CLARIANT) : EP 1 069 142, WO 02/44224, WO02/44225 WO02/44227 WO02/44229 WO02/44230 WO02/44231 WO02/44267 WO02/44268 WO02/44269 WO02/44270 WO02/44271 WO02/43677 WO02/43686 WO02/43687 WO02/43688 WO02/43689.

Les monomères hydrophobes à insaturation éthylénique de l'invention sont choisis de préférence parmi les acrylates ou les acrylamides de formule (1) suivante : 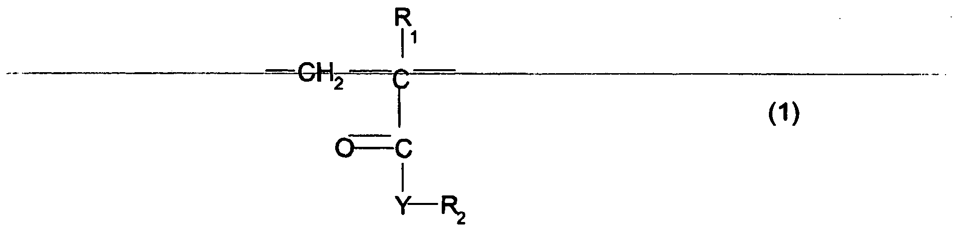 dans laquelle R₁ désigne un atome d'hydrogène, un radical alkyle linéaire ou ramifié en C₁-C₆ (de préférence méthyle) ; Y désigne O ou NH ; R₂ désigne un radical hydrophobe comportant une chaîne grasse ayant de 7 à 22 atomes de carbone, et de préférence de 7 à 18, et plus particulièrement de 12 à 18 atomes de carbone.

Le radical hydrophobe R₂ est choisi de préférence parmi les radicaux alkyles linéaires en C₇-C₁₈, saturés ou insaturés (par exemple n-octyle, n-décyle, n-hexadécyle, n-dodécyle, oléyle), ramifiés (par exemple isostéarique) ou cycliques (par exemple cyclododécane ou adamantane) ; les radicaux alkylperfluorés en C₇-C₁₈ (par exemple le groupement de formule -(CH₂)₂-(CF₂)₉-CF₃) ; le radical cholestéryle ou un ester de cholestérol comme l'hexanoate de cholestéryle ; les groupes polycycliques aromatiques comme le naphtalène ou le pyrène. Parmi ces radicaux, on préfère plus particulièrement les radicaux alkyles linéaires et ramifiés.

Selon une forme particulièrement préférée de l'invention, le radical hydrophobe R₂ comporte en plus au moins un motif oxyde d'alkylène et de préférence une chaîne polyoxyalkylénée. La chaîne polyoxyalkylénée de façon préférentielle est constituée de motifs oxyde d'éthylène et/ou de motifs oxyde de propylène et encore plus particulièrement constituée uniquement de motifs oxyde d'éthylène. Le nombre de moles de motifs oxyalkylénés varie en général de 1 à 30 moles et plus préférentiellement de 1 à 25 moles et encore plus préférentiellement de 3 à 20 moles.

Parmi ces polymères, on peut citer :
- les copolymères réticulés ou non réticulés, neutralisés ou non, comportant de 15 à 60 % en poids de motifs AMPS et de 40 à 85 % en poids de motifs (C₈-C₁₆)alkyl(méth)acrylamide ou de motifs (C₈-C₁₆)alkyl(méth)acrylate par rapport au polymère, tels que ceux décrits dans la demande EP-A-750 899 ;
- les terpolymères comportant de 10 à 90% en mole de motifs acrylamide, de 0,1 à 10% en mole de motifs AMPS et de 5 à 80% en mole de motifs n-(C₆-C₁₈)alkylacrylamide, par rapport au polymère, tels que ceux décrits dans le brevet US-A-5,089,578.

Comme polymères amphiphiles, on peut également citer les copolymères d'AMPS totalement neutralisé et de méthacrylate de n-dodécyle, de n-hexadécyle et/ou de n-octadécyle, ainsi que les copolymères d'AMPS et de n-dodécylméthacrylamide, non-réticulés et réticulés, tels que ceux décrits dans les articles de Morishima cités ci-dessus.

On citera plus particulièrement les copolymères amphiphiles réticulés ou non réticulés constitués :
(a) de motifs acide 2-acrylamido 2-méthylpropane sulfonique (AMPS) de formule (2) suivante : 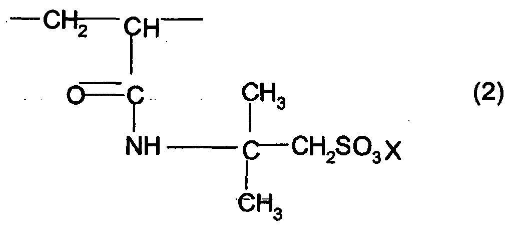 dans laquelle X⁺ est un proton, un cation de métal alcalin, un cation alcalino-terreux ou l'ion ammonium ;
(b) et de motifs de formule (3) suivante : 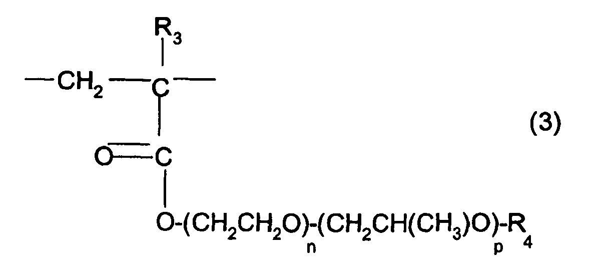
dans laquelle n et p, indépendamment l'un de l'autre désignent un nombre de moles et varie de 0 à 30, de préférence de 1 à 25 et plus préférentiellement de 3 à 20 sous réserve que n + p soit inférieur ou égal à 30, de préférence inférieur à 25 et encore mieux inférieur à 20 ; R₃ R₁ désigne un atome d'hydrogène, un radical alkyle linéaire ou ramifié en C₁-C₆ (de préférence méthyle) ; R₄ désigne un alkyle linéaire ou ramifié comportant m atomes de carbone allant de 7 à 22, de préférence de 7 à 18 atomes de carbone et encore mieux de 12 à 18 atomes de carbone.

Dans la formule (2), le cation X⁺ désigne plus particulièrement le sodium ou l'ammonium.

Parmi les monomères de formule (3) on peut citer :
- les esters d'acide (méth)acrylique et d'alcool gras en C₁₀-C₁₈ polyoxethylénés à 8 OE comme le produit GENAPOL C-080 vendu par la Société CLARIANT.
- les esters d'acide (méth)acrylique et d'oxoalcool gras en C₁₁ polyoxyéthyléné à 8 OE comme le produit GENAPOL UD-080 vendu par la Société CLARIANT.
- les esters d'acide (méth)acrylique et d'alcool gras polyoxyéthyléné en C₁₂-C₁₄ à 7 OE comme le produit GENAPOL LA-070 vendu par la Société CLARIANT.
- les esters d'acide (méth)acrylique et d'alcool gras polyoxyéthyléné en C₁₂-C₁₄ à 11 OE comme le produit GENAPOL LA-110 vendu par la Société CLARIANT.
- les esters d'acide (méth)acrylique et d'alcool gras polyoxyéthyléné en C₁₆-C₁₈ à 8 OE comme le produit GENAPOL T-080 vendu par la Société CLARIANT.
- les esters d'acide (méth)acrylique et d'alcool gras polyoxyéthyléné en C₁₆-C₁₈ à 15 OE comme le produit GENAPOL T-150 vendu par la Société CLARIANT.
- les esters d'acide (méth)acrylique et d'alcool gras polyoxyéthyléné en C₁₆-C₁₈ à 11 OE comme le produit GENAPOL T-110 vendu par la Société CLARIANT.
- les esters d'acide (méth)acrylique et d'alcool gras polyoxyéthyléné en C₁₆-C₁₈ à 20 OE comme le produit GENAPOL T-200 vendu par la Société CLARIANT.
- les esters d'acide (méth)acrylique et d'alcool gras polyoxyéthyléné en C₁₆-C₁₈ à 25 OE comme le produit GENAPOL T-250 vendu par la Société CLARIANT.
- les esters d'acide (méth)acrylique et d'alcool gras polyoxyéthyléné en C₁₈-C₂₂ à 25 OE et/ ou d'isoalcool gras polyoxyéthyléné en C₁₆-C₁₈ à 25 OE

On choisira plus particulièrement :
(i) ceux non réticulés pour lesquels p= 0, n = 7 ou 25, R₃ désigne méthyle et R₄ représente un mélange d'alkyle en C₁₂-C₁₄ ou en C₁₆-C₁₈ ,
(ii) ceux réticulés pour lesquels p = 0, n = 8 ou 25, R₃ désigne méthyle et R₄ représente un mélange d'alkyle en C₁₆-C₁₈,

Ces polymères sont décrits et synthétisés dans la demande EP1069142. Ces polymères amphiphiles particuliers peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le chlorhydrate de 2,2-azobis-[2-amidinopropane] (ABAH = 2,2-Azo-Bis-[2-Amidinopropane] Hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ces polymères amphiphiles peuvent être notamment obtenus par polymérisation radicalaire en milieu tert-butanol dans lequel ils précipitent. En utilisant la polymérisation par précipitation dans le tert-butanol, il est possible d'obtenir une distribution de la taille des particules du polymère particulièrement favorable pour ses utilisations.

La réaction peut être conduite à une température comprise entre 0 et 150°C, de préférence entre 10 et 100°C, soit à pression atmosphérique, soit sous pression réduite. Elle peut aussi être réalisée sous atmosphère inerte, et de préférence sous azote.

Les polymères conformes à l'invention de façon préférentielle sont neutralisés partiellement ou totalement par une base minérale ou organique telle que celles citées ci-dessus.

La concentration molaire en % des motifs de formule (2) et des motifs de formule (3) dans les polymères amphiphiles selon l'invention varie en fonction de l'application cosmétique souhaitée, de la nature de l'émulsion (huile-dans-eau ou eau-dans-huile) et des propriétés rhéologiques de la formulation recherchées. Elle peut varier entre 0,1 et 99,9 % en moles.

Les polymères d'AMPS amphiphiles selon l'invention peu hydrophobes seront plus appropriées pour l'épaississement et/ou la stabilisation des émulsions huile-dans-eau. La proportion molaire en motifs de formule (3) variera de préférence de 0,1 à 50 %, plus particulièrement de 1 à 25 % et encore plus particulièrement de 3 à 10%.

Les polymères d'AMPS amphiphiles selon l'invention plus hydrophobes seront plus appropriées pour l'épaississement et/ou la stabilisation des émulsions eau-dans-huile. La proportion molaire en motifs de formule (3) variera de préférence de 50,1 à 99,9 %, plus particulièrement de 60 à 95 % et encore plus particulièrement de 65 à 90 %.

La distribution des monomères dans les polymères de l'invention peut être, par exemple, alternée, bloc (y compris multibloc) ou quelconque.

Les polymères d'AMPS conformes à l'invention sont généralement présents dans des quantités en matière active allant de 0,01 à 20 % en poids, plus préférentiellement de 0,1 à 10 % en poids, encore plus préférentiellement de 0,1 à 5 % en poids et plus particulièrement encore de 0,5 à 2 % en poids de par rapport au poids total de la composition.

Les silicones comportant au moins un groupement polyoxyalkyléné monovalent terminal ou pendant utilisables selon l'invention sont hydrosolubles. C'est à dire qu'à la concentration de 0,2% à 25°C en poids dans l'eau elles forment une solution macroscopiquement homogène.

Les silicones hydrosolubles de l'invention sont de préférence choisies parmi les composés de formules générales (I) et (II) : 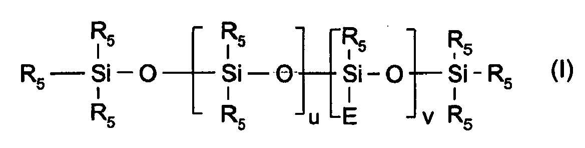 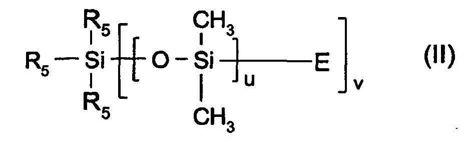 dans lesquelles :
- les radicaux R₅, identiques ou différents, désignent un radical hydrocarboné monovalent choisi parmi les groupes alkyle, aryle, aralkyle ne contenant pas plus de 10 atomes de carbone et de préférence choisis parmi les alkyles inférieurs en C₁-C₄ comme méthyle, éthyle, butyle ou bien choisis parmi phényle et benzyle et encore plus préférentiellement désignent tous méthyle ; certains des radicaux R₅ peuvent également contenir un plus un groupe éthylcyclohexylènemonooxyde et sont en faible proportion dans la chaîne polysiloxane ;
- u vaut 10 à 150, de préférence 25 à 100 et plus préférentiellement 65 à 85 ;
- v vaut 3 à 12, de préférence 4 à 10 et plus préférentiellement 5 à 8 ;
- E désigne un groupe -CₓH₂ₓ-(OC₂H₄)_{Y}-(OC₃H₆)_{z}-OR₆ où :
- x vaut 1 à 8 , de préférence de 2 à 4 et plus préférentiellement 3 ;
- y > 0 et z ≥ 0 ; y et z sont choisis de telle sorte que le poids total moléculaire du radical E varie de 200 à 10000 et plus préférentiellement de 350 à 4000 ;
- R₆ désigne hydrogène ; un radical alkyle en C₁-C₈, linéaire ou ramifié (de préférence en C₁-C₄ comme méthyle) ; un radical acyle en C₂-C₈ (de préférence en C₂-C₄ comme acétyle).

Dans la formule E, lorsque z est positif les unités oxyéthylène et oxypropylène peuvent distribuées de manière aléatoire dans la chaîne polyéther E et/ou sous forme de blocs.

Les silicones hydrosolubles conformes à l'invention sont connues et notamment décrites dans le brevet US 5,338,352 et leur mode de préparation est décrit notamment dans le brevet US 4,847,398.

De telles silicones sont par exemple vendues par la société OSI sous les dénominations commerciales Silwet L-720® , Silwet L-7002® , Silwet L-7600® , Silwet L-7604® , Silwet L-7605® , Silwet L-7607® , Silwet L-7657® , Silwet L-7200® , Silwet L7230® .

Selon l'invention, la ou les silicones hydrosolubles modifiées par des groupements oxyalkylènes peuvent représenter de 0,01 % à 20 % en poids, de préférence de 0,1 % à 15% en poids et plus particulièrement de 0,5 à 5% en poids par rapport au poids total de la composition finale.

Les compositions selon l'invention peuvent se présenter sous toutes les formes galéniques comportant une phase aqueuse classiquement utilisées pour une application topique et notamment les compositions sans phase grasse comme des lotions, des sérums ou des gels aqueux ; les compositions comprenant au moins une phase grasse comme des émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou des dispersions cire/phase aqueuse. Ces compositions sont préparées selon les méthodes usuelles.

Selon un mode particulier de réalisation de l'invention, les émulsions huile-dans-eau ou eau-dans huile préparées avec les polymères amphiphiles d'AMPS et les silicones polyoxyalkylénées hydrosolubles selon l'invention peuvent comporter seulement 1% en poids ou moins, et même être exemptes de tensioactifs émulsionnants, tout en étant stables au stockage.

La nature de la phase grasse rentrant dans la composition des émulsions selon l'invention n'est pas critique et elle peut ainsi être constituée par tous les composés qui sont déjà connus de façon générale comme convenant pour la fabrication d'émulsions de type eau dans huile. En particulier, ces composés peuvent être choisis, seuls ou en mélanges, parmi les différents corps gras, les huiles d'origine végétale, animale ou minérale, les cires naturelles ou synthétiques, et analogues.
Parmi les huiles pouvant rentrer dans la composition de la phase grasse, on peut notamment citer :
- les huiles minérales telles que l'huile de paraffine et l'huile de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle,
- les huiles synthétiques telles que l'huile de purcellin, le myristate de butyle, le myristate d'isopropyle, le myristate de cétyle, le palmitate d'isopropyle, l'adipate d'isopropyle, l'adipate d'éthylhéxyle, le stéarate de butyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle, les isoparaffines et les poly-a-oléfines.

Comme autres huiles utilisables dans les émulsions selon l'invention, on peut encore citer les benzoates d'alcools gras en C12-C15 (Finsolv TN de FINETEX), les alcools gras tels que l'alcool laurique, cétylique, myristique, stéarique, palmitique, oléique ainsi que le 2-octyldodécanol, les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés enC10-C18, les huiles fluorées et perfluorées, la lanoline, la lanoline hydrogénée, la lanoline acétylée et enfin les huiles de silicones, volatiles ou non.

Bien entendu, la phase grasse peut également contenir un ou plusieurs adjuvants cosmétiques lipophiles classiques, notamment ceux qui sont déjà utilisés de manière habituelle dans la fabrication et l'obtention des compositions cosmétiques antisolaires.

De manière classique, la phase aqueuse dispersante peut être constituée par de l'eau, ou un mélange d'eau et d'alcool(s) polyhydrique(s) comme par exemple le glycérol, le propylèneglycol et le sorbitol, ou bien encore un mélange d'eau et d'alcool(s) inférieur(s) hydrosoluble(s) tels que éthanol, isopropanol ou butanol (solution hydroalcoolique), et elle peut bien entendu en outre contenir des adjuvents cosmétiques classiques hydrosolubles.

Les compositions conformes à l'invention comportent un système filtrant les radiations UV pouvant comporter un ou plusieurs filtres UV organiques ou inorganiques actifs dans l'UVA et/ou l'UVB, hydrosolubles ou liposolubles ou bien insolubles dans les solvants cosmétiques couramment utilisés.

Les filtres organiques complémentaires sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénytacrytate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de benzoxazole tels que décrits dans les demandes de brevet EP0832642, EP1027883, EP1300137 et DE10162844 ; les dérivés de méthylène bis-(hydroxyphényl benzotriazole) tels que décrits dans s les demandes US 5,237,071, US 5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649, les 4,4-diarylbutadiènes tels que ceux décrits dans les demandes de brevet DE19755649 , EP916335, EP1133980, EP1133981 et EP-A-1008586 et leurs mélanges.

Comme exemples de filtres organiques actifs dans l'UV-A et/ou l'UV-B, on peut citer désignés ci-dessus sous leur nom INCI :
Dérivés de l'acide para-aminobenzoique:
   PABA,
   Ethyl PABA,
   Ethyl Dihydroxypropyl PABA,
   Ethylhexyl Diméthyl PABA vendu notamment sous le nom « ESCALOL 507 » par ISP, Glyceryl PABA,
   PEG-25 PABA vendu sous le nom « UVINUL P25 » par BASF,
Dérivés salicyliques:
   Homosalate vendu sous le nom « Eusolex HMS » par Rona/EM Industries,
   Ethylhexyl Salicylate vendu sous le nom « NEO HELIOPAN OS » par Haarmann et REIMER,
   Dipropyleneglycol Salicylate vendu sous le nom « DIPSAL » par SCHER,
   TEA Salicylate, vendu sous le nom « NEO HELIOPAN TS » par Haarmann et REIMER,
Dérivés du dibenzoylméthane :
   Butyl Methoxydibenzoylmethane vendu notamment sous le nom commercial « PARSOL 1789 » par HOFFMANN LAROCHE,
   Isopropyl Dibenzoylmethane,
Dérivés cinnamiques :
   Ethylhexyl Methoxycinnamate vendu notamment sous le nom commercial « PARSOL MCX » par HOFFMANN LAROCHE,
   Isopropyl Methoxy cinnamate,
   Isoamyl Methoxy cinnamate vendu sous le nom commercial « NEO HELIOPAN E 1000 » par HAARMANN et REIMER,
   Cinoxate,
   DEA Methoxycinnamate,
   - Diisopropyl Methylcinnamate,
   - Glyceryl Ethylhexanoate Dimethoxycinnamate
Dérivés de β,β-diphénylacrylate :
   Octocrylene vendu notamment sous le nom commercial « UVINUL N539 » par BASF,
   Etocrylene, vendu notamment sous le nom commercial « UVINUL N35 » par BASF,
Dérivés de la benzophénone :
   Benzophenone-1 vendu sous le nom commercial « UVINUL 400 » par BASF,
   Benzophenone-2 vendu sous le nom commercial « UVINUL D50 » par BASF
   Benzophenone-3 ou Oxybenzone, vendu sous le nom commercial « UVINUL M40 » par BASF,
   Benzophenone-4 vendu sous le nom commercial « UVINUL MS40 » par BASF, Benzophenone-5
   Benzophenone-6 vendu sous le nom commercial « Helisorb 11 » par Norquay
   Benzophenone-8 vendu sous le nom commercial « Spectra-Sorb UV-24 » par American Cyanamid
   Benzophenone-9 vendu sous le nom commercial« UVINUL DS-49» par BASF, Benzophenone-12,
   le 2-(4-diéthylamino-2-hydroxybenzoyl)-benzoate de n-hexyle
Dérivés du benzylidène camphre:
   3-Benzylidene camphor fabriqué sous le nom « MEXORYL SD» par CHIMEX,
   4-Methylbenzylidene camphor vendu sous le nom « EUSOLEX 6300 » par MERCK ,
   Benzylidene Camphor Sulfonic Acid fabriqué sous le nom « MEXORYL SL» par CHIMEX, Camphor Benzalkonium Methosulfate fabriqué sous le nom « MEXORYL SO » par CHIMEX,
   - Terephthalylidene Dicamphor Sulfonic Acid fabriqué sous le nom « MEXORYL SX » par CHIMEX,
   Polyacrylamidomethyl Benzylidene Camphor fabriqué sous le nom « MESORYL SW » par CHIMEX,
Dérivés de benzimidazole :
   Phenylbenzimidazole Sulfonic Acid vendu notamment sous le nom commercial « EUSOLEX 232 » par MERCK,
   Disodium Phenyl Dibenzimidazole Tetra-sulfonate vendu sous le nom commercial commercial « NEO HELIOPAN AP » par Haarmann et REIMER,
Dérivés de triazine :
   Anisotriazine vendu sous le nom commercial «TINOSORB S » par CIBA SPECIALTY CHEMICALS
   Ethylhexyl triazone vendu notamment sous le nom commercial «UVINUL T150 » par BASF,
   Diethylhexyl Butamido Triazone vendu sous le nom commercial « UVASORB HEB » par SIGMA 3V
   la 2,4,6- tris-(4'amino-benzalmalonate de diisobutyle)-s-triazine.
Dérivés de benzotriazole :
   Drometrizole Trisiloxane vendu sous le nom « Silatrizole » par RHODIA CHIMIE ,
   Methylène bis-Benzotriazolyl Tetramethylbutylphénol, vendu sous forme solide sous le nom commercial «MIXXIM BB/100 » par FAIRMOUNT CHEMICAL ou sous forme micronisé en dispersion aqueuse sous le nom commercial « TINOSORB M » par CIBA SPECIALTY CHEMICALS,
Dérivés anthraniliques :
   Menthyl anthranilate vendu sous le nom commercial commercial « NEO HELIOPAN MA » par Haarmann et REIMER,
Dérivés d'imidazolines :
   Ethylhexyl Dimethoxybenzylidene Dioxoimidazoline Propionate,
Dérivés de benzalmalonate :
   Polyorganosiloxane à fonctions benzalmalonate tel que le polysilicone-15 vendu sous la dénomination commerciale « PARSOL SLX » par HOFFMANN LAROCHE
4.4-diarylbutadiène :
   1,1-dicarboxy-(2'2'-dimethyl-propyi)-4,4-diphenylbutadiene
Dérivés de benzoxazole :
   2,4-bis-[5-1 (diméthylpropyl)benzoxazol-2-yl-(4-phenyl)-imino]-6-(2-ethylhexyl)-imino-1,3,5-triazine vendu sous le nom Uvasorb K2A par Sigma 3V ;
   et leurs mélanges.

Les filtres organiques plus particulièrement préférés sont choisis parmi les composés suivants :
Ethylhexyl Salicylate,
Ethylhexyl Methoxycinnamate
Octocrylene,
Butyl Methoxydibenzoylmethane
Phenylbenzimidazole Sulfonic Acid,
Benzophenone-3,
Benzophenone-4,
Benzophenone-5,
le 2-(4-diéthylamino-2-hydroxybenzoyl)-benzoate de n-hexyle 4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine Anisotriazine,
Ethylhexyl triazone,
Diethylhexyl Butamido Triazone,
Methylène bis-Benzotriazolyl Tetramethylbutylphénol
Drometrizole Trisiloxane
Polysilicone 15
1,1-dicarboxy-(2'2'-dimethyl-propyl)-4,4-diphenylbutadiene
2,4-bis-[5-1(diméthylpropyl)benzoxazol-2-yl-(4-phenyl)-imino]-6-(2-ethylhexyl)-imino-1,3,5-triazine et leurs mélanges.

Les filtres inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Le système filtrant selon l'invention est généralement présent dans les compositions selon l'invention à une teneur allant de 0,1 % à 30 % en poids et de préférence de 0,5 à 15 % , en poids, par rapport au poids total de la composition.

Les compositions selon l'invention peuvent également contenir des agents de bronzage et/ou de brunissage artificiels de la peau (agents autobronzants).

Les agents autobronzants sont généralement choisis parmi les composés mono ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342. On utilisera de préférence la DHA.

La DHA peut être utilisée sous forme libre et/ou encapsulée par exemple dans des vésicules lipidiques telle que des liposomes, notamment décrits dans la demande WO 97/25970.

Les agents autobronzants mono ou polycarbonylés sont généralement présents dans les compositions selon l'invention dans des proportions allant de 0,1 à 10% en poids par rapport au poids total de la composition, et de préférence de 0,2 à 8% en poids par rapport au poids total de la composition

Les compositions conformes à la présente invention peuvent comprendre en outre des adjuvants cosmétiques classiques notamment choisis parmi les solvants organiques, les épaississants ioniques ou non ioniques, les adoucissants, les humectants, les opacifiants, les stabilisants, les émollients, les silicones, les agents répulsifs contre les insectes, les parfums, les conservateurs, les tensioactifs, les charges, les actifs, les pigments, les polymères, les propulseurs, les agents alcalinisants ou acidifiants ou tout autre ingrédient habituellement utilisé dans le domaine cosmétique et/ou dermatologique.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Parmi les solvants organiques, on peut citer les alcools et polyols inférieurs.

Parmi les épaississants on peut citer les polymères acryliques réticulés comme les Carbomers fournis par Novéon, les polymères réticulés acrylates/C10-30 alkylacrylates du type Pemulen fournis par Novéon ou le polyacrylate-3 vendu sous le nom Viscophobe DB 1000 par Amerchol) ; les polymères dérivés de l'acide acrylamido 2-méthylpropane sulfonique (Hostacerin AMPS fourni par Clariant, Sépigel 305 fourni par Seppic), les polymères neutres synthétiques tels que la poly N-vinylpyrrolidone, les polysaccharides comme les gommes de guar, de xanthane et les dérivés cellulosiques modifiés ou non comme la gomme de guar hydroxypropylée, la méthylhydroxyéthylcellulose et l'hydroxypropylméthylcellulose.

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Les compositions cosmétiques selon l'invention peuvent par exemple être utilisées comme produit de soin et/ou de protection solaire pour le visage et/ou le corps. de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Les exemples concrets, mais nullement limitatifs, illustrant l'invention, vont maintenant être donnés.

### Exemples

On a réalisé et comparé :
Formule A : support cosmétique contenant un système photoprotecteur et un copolymère réticulé d'acide acrylamido-2-méthyl-2-propane-sulfonique (AMPS) et d'un monomère hydrophobe dérivé d'un alcool gras éthoxylé.
Formule B : un support cosmétique contenant un système photoprotecteur et un copolymère réticulé ou non réticulé d'acide acrylamido-2-méthyl-2-propane-sulfonique et d'un monomère hydrophobe dérivé d'un alcool gras éthoxylé associé à une polydiméthylsiloxane oxyalkylénée soluble dans l'eau.

| **Compositions testées** | **A (hors invention)** | **B (invention)** |
|---|---|---|
| Octocrylène (Uvinul N 539 de BASF) | 9 | 9 |
| Butyl Methoxydibenzoylméthane (Parsol 1789 de Hoffmann L aRoche) | 2.5 g | 2.5 g |
| Drometrizole Trisiloxane (Silatrizole de Rhodia) | 0.75g | 0.75g |
| C₁₂/C₁₅ Alkyl Benzoate | 6 g | 6 g |
| Copolymère réticulé d'AMPS et d'ester d'acide (méth)acrylique et d'alcool gras en C₁₆-C₁₈ polyoxyéthyléné à 25 OE (GENAPOL T-250) tel que celui décrit dans l'exemple 3 de la demande EP1069142 | 1.25 g | 1.25 g |
| Glycerol | 4 g | 4 g |
| Propylene glycol | 4 g | 4 g |
| Téréphtalylidene dicamphor sulfonic acid (Mexoryl SX de Chimex) | 1.5 g | 1.5 g |
| Oxyde de titane anatase (60 nm) enrobé silice/alumine en dispersion aqueuse protégée | 16.7 g | 16.7g |
| PDMS oxyalkylénée soluble dans l'eau ( Silwet L-7657® de OSI) | | 1g |
| Acide éthylène diamine tétracétique, sel disodique | qs | qs |
| Conservateurs | qs | qs |
| Eau | 53.2 g | 52.2 g |

Pour chacune des formulations A et B, on a ensuite déterminé in vivo le facteur de protection solaire (FPS).

La mesure du facteur de protection solaire a été effectuée selon la méthode suivante: on a appliqué ces formulations, à raison de 2 mg de produit/cm² de peau, sur le dos de 5 modèles humains, puis on a soumis simultanément les zones protégées et les zones non protégées de peau à l'action d'un simulateur solaire commercialisé sous le nom de "Xénon Multiport WG 320-UG 11" ; le facteur de protection solaire (FPS) a été alors calculé mathématiquement par le rapport du temps d'irradiation qui a été nécessaire pour atteindre le seuil érythématogène avec la formule A ou B (zone protégée) au temps qui a été nécessaire pour atteindre le seuil érythématogène sans formule (zone non protégée).

| | **Formule A (hors invention)** | **Formule B (invention)** |
|---|---|---|
| **FPS in vivo** | 16.5 ± 3.8 | 23 ± 3.9 |

On note qu'une composition photoprotectrice à base d'un copolymère réticulé ou non réticulé d'acide acrylamido-2-méthyl-2-propane-sulfonique et d'un monomère hydrophobe dérivé d'un alcool gras éthoxylé associé avec une polydiméthylsiloxane oxyalkylénée soluble dans l'eau permet d'augmenter le facteur de protection solaire de 28%.

## Revendications

1. Composition photoprotectrice comprenant au moins une phase aqueuse et de 0,1 % à 30 % en poids par rapport au poids total de la composition d'un système filtrant les radiations UV,**caractérisée par le fait qu'**elle contient :
(a) au moins un polymère amphiphile d'acide acrylamido 2-méthyl propane sulfonique, partiellement ou totalement neutralisé, réticulé ou non-réticulé; ledit polymère comprenant à la fois une partie hydrophile et une partie hydrophobe comportant au moins une chaîne hydrocarbonée comportant au moins 7 atomes de carbone ;
(b) au moins une silicone hydrosoluble comportant au moins un groupement polyoxyalkyléné monovalent terminal ou pendant.

2. Composition selon la revendication 1, **caractérisée en ce que** le polymère d'AMPS est neutralisé partiellement ou totalement par une base minérale ou organique.

3. Composition selon la revendication 2, **caractérisée en ce que** la base minérale est choisie parmi la soude, potasse ou ammoniaque.

4. Composition selon la revendication 2, **caractérisée en ce que** la base organique est choisie parmi la mono-, di- ou tri-éthanolamine, l'aminométhylpropanediol, la N-méthyl-glucamine, les acides aminés basiques et leurs mélanges.

5. Composition selon l'une quelconque des revendications 1 à 4, où le polymère d'AMPS est neutralisé à au moins 90 %.

6. Composition selon l'une quelconque des revendications 1 à 5, où le polymère d'AMPS est réticulé et où l'agent de réticulation est choisi parmi les composés à polyinsaturation oléfinique couramment utilisés pour la réticulation des polymères obtenus par polymérisation radicalaire.

7. Composition selon la revendication 6, où l'agent de réticulation est choisi parmi le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate d'éthylèneglycol ou de tétraéthylèneglycol, le triméthylol propane triacrylate, le methylène-bis-acrylamide, le méthylène-bis-méthacrylamide, la triallylamine, le triallylcyanurate, le diallylmaléate, la tétraallyléthylènediamine, le tétraallyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou leurs mélanges.

8. Composition selon la revendication 6, où l'agent de réticulation est choisi parmi le méthylène-bis-acrylamide, le méthacrylate d'allyle ou le triméthylol propane triacrylate (TMPTA).

9. Composition selon l'une quelconque des revendications 7 à 8, où le taux de réticulation va en général de 0,01 à 10 % en mole et plus particulièrement de 0,2 à 2 % en mole par rapport au polymère.

10. Composition selon l'une quelconque des revendications 1 à 9, où le polymère d'AMPS amphiphile comporte au moins une chaîne grasse comportant de 7 à 30 atomes de carbone, plus préférentiellement de 7 à 22 atomes de carbone et encore plus préférentiellement de 7 à 18 atomes et plus particulièrement de 12 à 18 atomes de carbones.

11. Composition selon l'une quelconque des revendications 1 à 10, où le polymère d'AMPS amphiphile a un poids moléculaire en poids allant de 50 000 à 10 000 000, plus préférentiellement de 100 000 à 8 000 000 et encore plus préférentiellement de 100 000 à 7 000 000.

12. Composition selon l'une quelconque des revendications 1 à 11, où les polymères d'AMPS amphiphiles sont choisis parmi les polymères amphiphiles statistiques d'AMPS modifiés par réaction avec une n-monoalkylamine ou une di-n-alkylamine en C₆-C₂₂ et pouvant contenir un ou plusieurs monomères hydrophiles à insaturation éthylénique.

13. Composition selon l'une quelconque des revendications 1 à 12, où les polymères d'AMPS amphiphiles sont choisis parmi les polymères d'AMPS et d'au moins un monomère à insaturation éthylénique comportant au moins une partie hydrophobe ayant de 7 à 30 atomes de carbone et plus préférentiellement de 7 à 22 atomes de carbone et encore plus préférentiellement de 7 à 18 atomes de carbone et plus particulièrement de 12 à 18 atomes de carbone et éventuellement un ou plusieurs co-monomères hydrophiles à insaturation éthylènique.

14. Composition selon la revendication 13, où les monomères à insaturation éthylénique comportant au moins une partie hydrophobe ayant de 7 à 30 atomes de carbone sont choisis parmi les acrylates ou les acrylamides de formule (1) suivante : 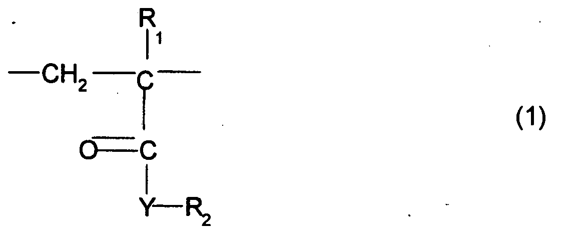 dans laquelle R₁ désigne un atome d'hydrogène, un radical alkyle linéaire ou ramifié en C₁-C₆ (de préférence méthyle) ; Y désigne O ou NH ; R₂ désigne un radical hydrophobe comportant une chaîne grasse ayant de 7 à 22 atomes de carbone, et de préférence de 7 à 18, et plus particulièrement de 12 à 18 atomes de carbone.

15. Composition selon la revendication 20, où le radical hydrophobe R₂ est choisi de parmi les radicaux alkyles en C₇-C₁₈ linéaires ou ramifiés, saturés ou insaturés les radicaux alkylperfluorés en C₇-C₁₈ ; le radical cholestéryle ou un ester de cholestérol ; les groupes polycycliques aromatiques.

16. Composition selon la revendication 14 ou 15, où le radical hydrophobe R₂ comporte en plus au moins un motif oxyde d'alkylène et de préférence une chaîne polyoxyalkylénée.

17. Composition selon la revendication 16, où le nombre de moles de motifs oxyalkylénés varie de 1 à 30 moles et plus préférentiellement de 1 à 25 moles et encore plus préférentiellement de 3 à 20 moles.

18. Composition selon l'une quelconque des revendications 14 à 17, où les polymères d'AMPS amphiphiles sont des copolymères amphiphiles constitués :
(a) de motifs acide 2-acrylamido 2-méthylpropane sulfonique (AMPS) de formule (2) suivante : 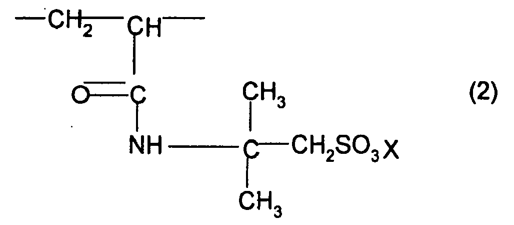 dans laquelle X⁺ est un proton, un cation de métal alcalin, un cation alcalino-terreux ou l'ion ammonium ;
(b) et de motifs de formule (3) suivante : 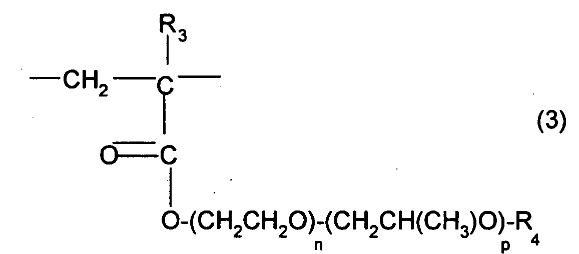 dans laquelle n et p, indépendamment l'un de l'autre désignent un nombre de moles et varie de 0 à 30, de préférence de 1 à 25 et plus préférentiellement de 3 à 20 sous réserve que n + p soit inférieur ou égal à 30, de préférence inférieur à 25 et encore mieux inférieur à 20 ; R₃ désigne un atome d'hydrogène, un radical alkyle linéaire ou ramifié en C₁-C₆ (de préférence méthyle) ; R₄ désigne un alkyle linéaire ou ramifié comportant m atomes de carbone allant de 7 à 22, de préférence de 7 à 18 atomes de carbone et encore mieux de 12 à 18 atomes de carbone.

19. Composition selon la revendication 18, où X⁺ désigne le sodium ou l'ammonium.

20. Composition selon la revendication 17 ou 18, où le motif de formule (3) est choisi parmi :
- les esters d'acide (méth)acrylique et d'alcool gras en C₁₀-C₁₈ polyoxethylénés à 8 OE ;
- les esters d'acide (méth)acrylique et d'oxoalcool gras en C₁₁ polyoxyéthyléné à 8 OE ;
- les esters d'acide (méth)acrylique et d'alcool gras polyoxyéthyléné en C₁₂-C₁₄ à 7 OE ;
- les esters d'acide (méth)acrylique et d'alcool gras polyoxyéthyléné en C₁₂-C₁₄ à 11 OE ;
- les esters d'acide (méth)acrylique et d'alcool gras polyoxyéthyléné en C₁₆-C₁₈ à 8 OE ;
- les esters d'acide (méth)acrylique et d'alcool gras polyoxyéthyléné en C₁₆-C₁₈ à 15 OE ;
- les esters d'acide (méth)acrylique et d'alcool gras polyoxyéthyléné en C₁₆-C₁₈ à 11 OE ;
- les esters d'acide (méth)acrylique et d'alcool gras polyoxyéthyléné en C₁₆-C₁₈ à 20 OE ;
- les esters d'acide (méth)acrylique et d'alcool gras polyoxyéthyléné en C₁₆-C₁₈ à 25 OE ;
- les esters d'acide (méth)acrylique et d'alcool gras polyoxyéthyléné en C₁₈-C₂₂ à 25 OE et/ ou d'isoalcool gras polyoxyéthyléné en C₁₆-C₁₈ à 25 OE

21. Composition selon l'une quelconque des revendications 14 à 20, où les polymères d'AMPS amphiphiles sont chosis parmi
(i) ceux non réticulés pour lesquels p= 0, n = 7 ou 25, R₃ désigne méthyle et R₄ représente un mélange d'alkyle en C₁₂-C₁₄ ou en C₁₆-C₁₈,
(ii) ceux réticulés pour lesquels p = 0, n = 8 ou 25, R₃ désigne méthyle et R₄ représente un mélange d'alkyle en C₁₆-C₁₈.

22. Composition selon l'une quelconque des revendications 18 à 21, où la proportion molaire en motifs de formule (3) varie de 0,1 à 50 %, plus particulièrement de 1 à 25 % et encore plus particulièrement de 3 à 10%.

23. Composition selon l'une quelconque des revendications 18 à 21, où la proportion molaire en motifs de formule (3) varie de 50,1 à 99,9 % et plus particulièrement de 60 à 95 % et encore plus particulièrement de 65 à 90 %.

24. Composition selon l'une quelconque des revendications 1 à 23, où les polymères d'AMPS sont présents dans des quantités en matière active allant de 0,01 à 20 % en poids, plus préférentiellement de 0,1 à 10 % en poids, encore plus préférentiellement de 0,1 à 5 % en poids et plus particulièrement encore de 0,5 à 2 % en poids de par rapport au poids total de la composition.

25. Composition selon l'une quelconque des revendications 1 à 24, où la silicone hydrosoluble comportant au moins un groupement polyoxyalkyléné monovalent terminal ou pendant est choisie parmi les composés de formules générales (I), (II) suivantes : 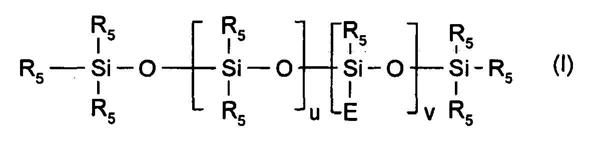 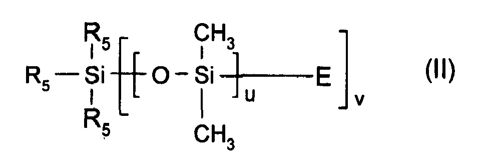 dans lesquelles :
- les radicaux R₅, identiques ou différents, désignent un radical hydrocarboné monovalent choisi parmi les groupes alkyle, aryle, aralkyle ne contenant pas plus de 10 atomes de carbone et de préférence choisis parmi les alkyles inférieurs en C₁-C₄ ; certains des radicaux R₅ peuvent contenir en plus un groupe éthylcyclohexylènemonooxyde et sont en faible proportion dans la chaîne polysiloxane ;
- u vaut 10 à 150, de préférence 25 à 100 et plus préférentiellement 65 à 85 ;
- v vaut 3 à 12, de préférence 4 à 10 et plus préférentiellement 5 à 8 ;
- E désigne un groupe -CₓH₂ₓ-(OC₂H₄)_{Y}-(OC₃H₆)_{Z}-OR₆ où :
- x vaut 1 à 8;
- y > 0 et z ≥ 0; y et z sont choisis de telle sorte que le poids total moléculaire du radical E varie de 200 à 10000;
- R₆ désigne hydrogène ; un radical alkyle en C₁-C₈. linéaire ou ramifié ; un radical acyle en C₂-C₈, linéaire ou ramifié.

26. Composition selon la revendication 27, où les R₅ désignent un alkyle inférieur en C₁-C_{4 ;} R₆ désigne hydrogène, un radical alkyle en C₁-C₄ ou un radical acyle C₂-C₄ ; x vaut 2 à 4 et y et z sont choisis de telle sorte que le poids total moléculaire du radical E varie de 350 à 4000.

27. Composition selon la revendication 26, où tous les radicaux R₅ désignent méthyle ; R₆ désigne hydrogène, méthyle ou acétyle ; x vaut 3.

28. Composition selon l'une quelconque des revendications 1 à 27, où la silicone hydrosoluble comportant au moins un groupement polyoxyalkyléné monovalent terminal ou pendant est présent dans des concentrations allant de 0,01 % à 20 % en poids, de préférence de 0,1 % à 15% en poids et plus particulièrement de 0,5 à 5 % en poids par rapport au poids total de la composition.

29. Composition selon l'une quelconque des revendications 1 à 28, **caractérisée en ce que** le système filtrant les radiation UV contient au moins un filtre solaire organique ou inorganique complémentaire actif dans l'UV-A et/ou l'UV-B, hydrosoluble, liposoluble ou bien insoluble dans les solvants cosmétiques couramment utilisés.

30. Composition selon la revendication 29, où les filtres organiques sont choisis parmi les anthranilates ; les dérivés cinnamiques ; les dérivés de dibenzoylméthane ; les dérivés salicyliques, les dérivés du camphre ; les dérivés de triazine ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de benzoxazole ; les dérivés de méthylène bis-(hydroxyphényl benzotriazole) ; les polymères filtres et silicones filtres ; les dimères dérivés d'α-alkylstyrène, les 4,4-diarylbutadiène et leurs mélanges.

31. Composition selon la revendication 30, ou les filtres organiques sont choisis parmi
Ethylhexyl Salicylate,
Ethylhexyl Methoxycinnamate
Octocrylene,
Butyl Methoxydibenzoylmethane
Phenylbenzimidazole Sulfonic Acid,
Benzophenone-3,
Benzophenone-4,
Benzophenone-5,
le 2-(4-diéthylamino-2-hydroxybenzoyl)-benzoate de n-hexyle
4-Methylbenzylidene camphor,
Terephthalylidene Dicamphor Sulfonic Acid,
Disodium Phenyl Dibenzimidazole Tetra-sulfonate,
la 2,4,6-tris-(4'-amino benzalmalonate de diisobutyle)-s-triazine
Anisotriazine,
Ethylhexyl triazone,
Diethylhexyl Butamido Triazone,
Méthylène bis-Benzotriazolyl Tetramethylbutylphénol
Drometrizole Trisiloxane
Polysilicone-15
1,1-dicarboxy-(2'2'-dimethyl-propyl)-4,4-diphenylbutadiene
2,4-bis-[5-1(diméthylpropyl)benzoxazol-2-yl-(4-phenyl)-imino]-6-(2-ethylhexyl)-imino-1,3,5-triazine et leurs mélanges.

32. Composition selon la revendication 29, où les filtres inorganiques sont choisis parmi des pigments ou des nanopigments d'oxydes métalliques, enrobés ou non.

33. Composition selon la revendication 32, où les filtres complémentaires inorganiques sont des nanopigments d'oxyde de titane, amorphe ou cristallisé, sous forme rutile et/ou anatase, de fer, de zinc, de zirconium ou de cérium.

34. Composition selon l'une quelconque des revendications 1 à 33 **caractérisée en ce qu'**elle contient en outre au moins un agent de bronzage et/ou de brunissage artificiel de la peau.

35. Composition selon l'une quelconque des revendications 1 à 34, **caractérisée en ce qu'**elle contient en outre au moins un adjuvant cosmétique choisi parmi les solvants organiques, les épaississants ioniques ou non ioniques, les adoucissants, les humectants, les opacifiants, les stabilisants, les émollients, les silicones, les agents répulsifs contre les insectes, les parfums, les conservateurs, les tensioactifs, les charges, les pigments, les polymères, les propulseurs, les agents alcalinisants ou acidifiants ou tout autre ingrédient habituellement utilisé dans le domaine cosmétique et/ou dermatologique.

36. Composition selon l'une quelconque des revendications 1 à 35, **caractérisée en ce qu'**elle se présente sous forme de lotion ou sérum, de gel aqueux, d'émulsion huile-dans-eau ou eau-dans-huile ; d'émulsions multiples, de microémulsions, de dispersions vésiculaires de type ionique et/ou non ionique ou des dispersion cire/phase aqueuse.

37. Composition selon l'une quelconque des revendications 1 à 36, **caractérisée en ce qu'**elle se présente sous forme d'émulsion huile-dans-eau ou eau-dans huile comportant au plus 1% en poids par rapport au poids total de la composition en tensioactif émulsionnant.

38. Utilisation d'une composition telle que définie selon l'une quelconque des revendications 1 à 37 pour la fabrication de produits pour le traitement cosmétique de la peau, des lèvres et des cheveux, y compris le cuir chevelu, en particulier pour la protection et/ou le soin de la peau, des lèvres et/ou des cheveux et/ou pour le maquillage de la peau et/ou des lèvres.

39. Utilisation de l'association d'au moins un polymère amphiphile d'acide acrylamido 2-méthyl propane sulfonique, comportant au moins une chaîne grasse partiellement ou totalement neutralisé, réticulé ou non-réticulé et d'au moins une silicone hydrosoluble comportant au moins un groupement polyoxyalkyléné monovalent terminal ou pendant tels que définis dans l'une quelconque des revendications précédentes, dans une composition cosmétique aqueuse comprenant de 0,1 % à 30 % en poids par rapport au poids total de la composition d'un système photoprotecteur capable de filtrer le rayonnement UV, pour augmenter le facteur de protection solaire.

## Claims

1. Photoprotective composition comprising at least one aqueous phase and from 0.1% to 30% by weight, relative to the total weight of the composition, of a system for screening out UV radiation, **characterized in that** it contains:
(a) at least one partially or totally neutralized, crosslinked or non-crosslinked amphiphilic polymer of acrylamido-2-methylpropanesulphonic acid; said polymer comprising both a hydrophilic portion and a hydrophobic portion comprising at least one hydrocarbon-based chain containing at least 7 carbon atoms;
(b) at least one water-soluble silicone comprising at least one terminal or pendent monovalent polyoxyalkylene group.

2. Composition according to Claim 1, **characterized in that** the AMPS polymer is partially or totally neutralized with an inorganic or organic base.

3. Composition according to Claim 2, **characterized in that** the inorganic base is chosen from sodium hydroxide, potassium hydroxide and aqueous ammonia.

4. Composition according to Claim 2, **characterized in that** the organic base is chosen from monoethanolamine, diethanolamine, triethanolamine, aminomethylpropane-diol, N-methylglucamine and basic amino acids, and mixtures thereof.

5. Composition according to any one of Claims 1 to 4, in which the AMPS polymer is at least 90% neutralized.

6. Composition according to any one of Claims 1 to 5, in which the AMPS polymer is crosslinked and in which the crosslinking agent is chosen from the polyolefinically unsaturated compounds commonly used for crosslinking polymers obtained by free-radical polymerization.

7. Composition according to Claim 6, in which the crosslinking agent is chosen from divinylbenzene, diallyl ether, dipropylene glycol diallyl ether, polyglycol diallyl ethers, triethylene glycol divinyl ether, hydroquinone diallyl ether, ethylene glycol di(meth)acrylate or tetraethylene glycol di(meth)acrylate, trimethylolpropane triacrylate, methylenebisacrylamide, methylenebismethacrylamide, triallylamine, triallyl cyanurate, diallyl maleate, tetraallylethylenediamine, tetraallyloxyethane, trimethylolpropane diallyl ether, allyl (meth)acrylate, allylic ethers of alcohols of the sugar series, or other allyl or vinyl ethers of polyfunctional alcohols, and also allylic esters of phosphoric and/or vinyl phosphonic acid derivatives, or mixtures thereof.

8. Composition according to Claim 6, in which the crosslinking agent is chosen from methylenebisacrylamide, allyl methacrylate or trimethylolpropane triacrylate (TMPTA).

9. Composition according to either one of Claims 7 and 8, in which the degree of crosslinking ranges in general from 0.01 mol% to 10 mol%, and more particularly from 0.2 mol% to 2 mol% relative to the polymer.

10. Composition according to any one of Claims 1 to 9, in which the amphiphilic AMPS polymer comprises at least one fatty chain containing from 7 to 30 carbon atoms, more preferably from 7 to 22 carbon atoms, even more preferably from 7 to 18 carbon atoms, and more particularly from 12 to 18 carbon atoms.

11. Composition according to any one of Claims 1 to 10, in which the amphiphilic AMPS polymer has a weight-average molecular weight ranging from 50 000 to 10 000 000, more preferably from 100 000 to 8 000 000, and even more preferably from 100 000 to 7 000 000.

12. Composition according to any one of Claims 1 to 11, in which the amphiphilic AMPS polymers are chosen from random amphiphilic polymers of AMPS modified by reaction with a C₆-C₂₂ n-monoalkylamine or di-n-alkylamine and which may contain one or more ethylenically unsaturated hydrophilic monomers.

13. Composition according to any one of Claims 1 to 12, in which the amphiphilic AMPS polymers are chosen from polymers of AMPS and of at least one ethylenically unsaturated monomer comprising at least one hydrophobic portion containing from 7 to 30 carbon atoms, more preferably from 7 to 22 carbon atoms, even more preferably from 7 to 18 carbon atoms, and more particularly from 12 to 18 carbon atoms, and optionally one or more ethylenically unsaturated hydrophilic comonomers.

14. Composition according to Claim 13, in which the ethylenically unsaturated monomers comprising at least one hydrophobic portion containing from 7 to 30 carbon atoms are chosen from the acrylates or acrylamides of formula (1) below: 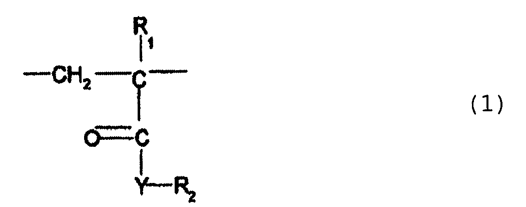 in which R₁ denotes a hydrogen atom or a linear or branched C₁-C₆ alkyl radical (preferably methyl); Y denotes O or NH; R₂ denotes a hydrophobic radical comprising a fatty chain containing from 7 to 22 carbon atoms, preferably from 7 to 18, and more particularly from 12 to 18 carbon atoms.

15. Composition according to Claim 20, in which the hydrophobic radical R₂ is chosen from linear or branched, saturated or unsaturated C₇-C₁₈ alkyl radicals; C₇-C₁₈ alkylperfluoro radicals; the cholesteryl radical or a cholesterol ester; aromatic polycyclic groups.

16. Composition according to Claim 14 or 15, in which the hydrophobic radical R₂ also comprises at least one alkylene oxide unit, and preferably a polyoxyalkylene chain.

17. Composition according to Claim 16, in which the number of moles of oxyalkylene units ranges from 1 to 30 mol, more preferably from 1 to 25 mol, and even more preferably from 3 to 20 mol.

18. Composition according to any one of Claims 14 to 17, in which the amphiphilic AMPS polymers are amphiphilic copolymers constituted:
(a) of 2-acrylamido-2-methylpropanesulphonic acid (AMPS) units of formula (2) below: 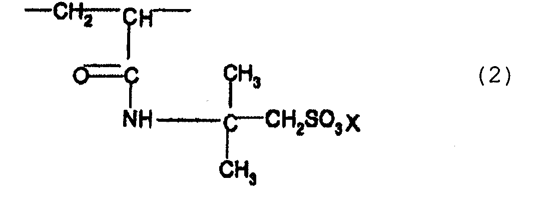 in which X⁺ is a proton, an alkali metal cation, an alkaline-earth metal cation or an ammonium ion;
(b) and of units of formula (3) below: 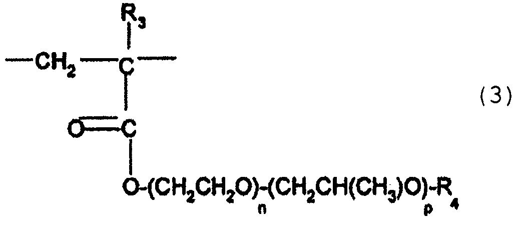 in which n and p, independently of one another, denote a number of moles and range from 0 to 30, preferably from 1 to 25, and more preferably from 3 to 20, with the proviso that n + p is less than or equal to 30, preferably less than 25, and better still less than 20; R₃ denotes a hydrogen atom or a linear or branched C₁-C₆ alkyl radical (preferably methyl); R₄ denotes a linear or branched alkyl containing m carbon atoms ranging from 7 to 22, preferably from 7 to 18 carbon atoms, and better still from 12 to 18 carbon atoms.

19. Composition according to Claim 18, in which X⁺ denotes sodium or ammonium.

20. Composition according to Claim 17 or 18, in which the unit of formula (3) is chosen from:
- esters of (meth) acrylic acid and of a C₁₀-C₁₈ fatty alcohol polyoxyethylenated with 8 EO;
- esters of (meth)acrylic acid and of a C₁₁ fatty oxoalcohol polyoxyethylenated with 8 EO;
- esters of (meth) acrylic acid and of a C₁₂-C₁₄ polyoxyethylenated fatty alcohol with 7 EO;
- esters of (meth)acrylic acid and of a C₁₂-C₁₄ polyoxyethylenated fatty alcohol with 11 EO;
- esters of (meth) acrylic acid and of a C₁₆-C₁₈ polyoxyethylenated fatty alcohol with 8 EO;
- esters of (meth) acrylic acid and of a C₁₆-C₁₈ polyoxyethylenated fatty alcohol with 15 EO;
- esters of (meth) acrylic acid and of a C₁₆-C₁₈ polyoxyethylenated fatty alcohol with 11 EO;
- esters of (meth) acrylic acid and of a C₁₆-C₁₈ polyoxyethylenated fatty alcohol with 20 EO;
- esters of (meth) acrylic acid and of a C₁₆-C₁₈ polyoxyethylenated fatty alcohol with 25 EO;
- esters of (meth) acrylic acid and of a C₁₈-C₂₂ polyoxyethylenated fatty alcohol with 25 EO and/or of a C₁₆-C₁₈ polyoxyethylenated fatty isoalcohol with 25 EO.

21. Composition according to any one of Claims 14 to 20, in which the amphiphilic AMPS polymers are chosen from:
(i) the non-crosslinked products for which p = 0, n = 7 or 25, R₃ denotes methyl and R₄ represents a C₁₂-C₁₄ or C₁₆-C₁₈ alkyl mixture,
(ii) the crosslinked products for which p = 0, n = 8 or 25, R₃ denotes methyl and R₄ represents a C₁₆-C₁₈ alkyl mixture.

22. Composition according to any one of Claims 18 to 21, in which the molar proportion of units of formula (3) ranges from 0.1% to 50%, more particularly from 1% to 25%, and even more particularly from 3% to 10%.

23. Composition according to any one of Claims 18 to 21, in which the molar proportion of units of formula (3) ranges from 50.1% to 99.9%, more particularly from 60% to 95%, and even more particularly from 65% to 90%.

24. Composition according to any one of Claims 1 to 23, in which the AMPS polymers are present in active material amounts ranging from 0.01% to 20% by weight, more preferably from 0.1% to 10% by weight, even more preferably from 0.1% to 5% by weight, and even more particularly from 0.5% to 2% by weight relative to the total weight of the composition.

25. Composition according to any one of Claims 1 to 24, in which the water-soluble silicone comprising at least one terminal or pendent monovalent polyoxyalkylene group is chosen from the compounds of general formula (I) and (II) below: 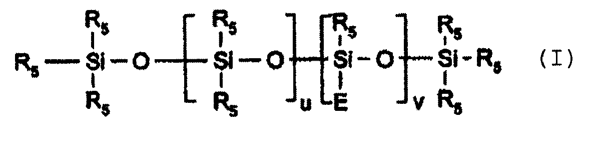 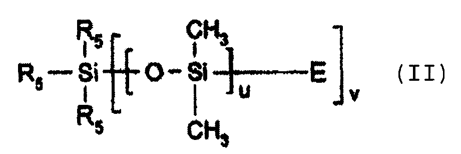 in which:
- the radicals R₅, which may be identical or different, denote a monovalent hydrocarbon-based radical chosen from alkyl, aryl and aralkyl groups containing not more than 10 carbon atoms and preferably chosen from C₁-C₄ lower alkyls; some of the radicals R₅ may also contain an ethylcyclohexylene monoxide group and are in small proportion in the polysiloxane chain;
- u is from 10 to 150, preferably from 25 to 100, and more preferably from 65 to 85;
- v is from 3 to 12, preferably from 4 to 10, and more preferably from 5 to 8;
- E denotes a -CₓH₂ₓ-(OC₂H₄)_{y}-(OC₃H₆)_{z}-OR₆ group in which:
- x is from 1 to 8;
- y > 0 and z ≥ 0; y and z are chosen such that the total molecular weight of the radical E ranges from 200 to 10 000;
- R₆ denotes hydrogen; a linear or branched C₁-C₈ alkyl radical; or a linear or branched C₂-C₈ acyl radical.

26. Composition according to Claim 27, in which the R₅ denote a C₁-C₄ lower alkyl; R₆ denotes hydrogen, a C₁-C₄ alkyl radical or a C₂-C₄ acyl radical; x is from 2 to 4 and y and z are chosen such that the total molecular weight of the radical E ranges from 350 to 4000.

27. Composition according to Claim 26, in which all the radicals R₅ denote methyl; R₆ denotes hydrogen, methyl or acetyl; and x is 3.

28. Composition according to any one of Claims 1 to 27, in which the water-soluble silicone comprising at least one terminal or pendent monovalent polyoxyalkylene group is present in concentrations ranging from 0.01% to 20% by weight, preferably from 0.1% to 15% by weight, and more particularly from 0.5% to 5% by weight relative to the total weight of the composition.

29. Composition according to any one of Claims 1 to 28, **characterized in that** the system for screening out UV radiation contains at least one supplementary organic or inorganic sunscreen agent active in the UV-A and/or the UV-B range, which is water-soluble, liposoluble or insoluble in the cosmetic solvents commonly used.

30. Composition according to Claim 29, in which the organic screening agents are chosen from anthranilates; cinnamic derivatives; dibenzoylmethane derivatives; salicylic derivatives; camphor derivatives; triazine derivatives; benzophenone derivatives; β,β-diphenyl-acrylate derivatives; benzotriazole derivatives; benzalmalonate derivatives; benzimidazole derivatives; imidazolines; bisbenzoazolyl derivatives; p-aminobenzoic acid (PABA) derivatives; benzoxazole derivatives; methylenebis(hydroxyphenylbenzotriazole) derivatives; screening polymers and screening silicones; dimers derived from α-alkylstyrene; and 4,4-diarylbutadiene, and mixtures thereof.

31. Composition according to Claim 30, in which the organic screening agents are chosen from:
- Ethylhexyl salicylate,
- Ethylhexyl methoxycinnamate,
- Octocrylene,
- Butyl methoxydibenzoylmethane,
- Phenylbenzimidazolesulphonic acid,
- Benzophenone-3,
- Benzophenone-4,
- Benzophenone-5,
- n-Hexyl 2-(4-diethylamino-2-hydroxybenzoyl)benzoate,
- 4-Methylbenzylidenecamphor,
- Terephthalylidenedicamphorsulphonic acid,
- Disodium phenyldibenzimidazoletetrasulphonate,
- 2,4,6-Tris(diisobutyl 4'-aminobenzalmalonate)-s-triazine,
- Anisotriazine,
- Ethylhexyl triazone,
- Diethylhexyl butamido triazone,
- Methylene bis(benzotriazolyl)tetramethylbutylphenol,
- Drometrizole trisiloxane,
- Polysilicone-15,
- 1,1-Dicarboxy(2',2'-dimethylpropyl)-4,4-diphenyl-butadiene,
- 2,4-bis[5-1(Dimethylpropyl)benzoxazol-2-yl(4-phenyl)-imino]-6-(2-ethylhexyl)imino-1,3,5-triazine, and
mixtures thereof.

32. Composition according to Claim 29, in which the inorganic screening agents are chosen from coated or uncoated metal oxide pigments or nanopigments.

33. Composition according to Claim 32, in which the supplementary inorganic screening agents are nanopigments of titanium oxide, amorphous or crystallized in rutile and/or anatase form, or of iron oxide, zinc oxide, zirconium oxide or cerium oxide.

34. Composition according to any one of Claims 1 to 33, **characterized in that** it also contains at least one agent for artificially tanning and/or browning the skin.

35. Composition according to any one of Claims 1 to 34, **characterized in that** it also contains at least one cosmetic adjuvant chosen from organic solvents, ionic or non-ionic thickeners, demulcents, humectants, opacifiers, stabilizers, emollients, silicones, insect repellents, fragrances, preservatives, surfactants, fillers, pigments, polymers, propellants, basifying or acidifying agents or any other ingredient customarily used in the cosmetics and/or dermatology field.

36. Composition according to any one of Claims 1 to 35, **characterized in that** it is in the form of a lotion or serum, an aqueous gel, an oil-in-water or water-in-oil emulsion; multiple emulsions, microemulsions, vesicular dispersions of ionic and/or non-ionic type or wax/aqueous phase dispersions.

37. Composition according to any one of Claims 1 to 36, **characterized in that** it is in the form of an oil-in-water or water-in-oil emulsion comprising not more than 1% by weight of emulsifying surfactant, relative to the total weight of the composition.

38. Use of a composition as defined in any one of Claims 1 to 37, for the manufacture of products for the cosmetic treatment of the skin, the lips and the hair, including the scalp, in particular for protecting and/or caring for the skin, the lips and/or the hair and/or for making up the skin and/or the lips.

39. Use of the combination of at least one partially or totally neutralized, crosslinked or non-crosslinked amphiphilic polymer of acrylamido-2-methylpropanesulphonic acid comprising at least one fatty chain and of at least one water-soluble silicone comprising at least one terminal or pendent monovalent polyoxyalkylene group, as defined in any one of the preceding claims, in an aqueous cosmetic composition comprising from 0.1% to 30% by weight, relative to the total weight of the composition, of a photoprotective system capable of screening out UV radiation, for increasing the sun protection factor.

## Patentansprüche

1. Lichtschutzzusammensetzung, die mindestens eine wässrige Phase und, bezogen auf das Gesamtgewicht der Zusammensetzung, 0,1 bis 30 Gew.-% eines die UV-Strahlung filternden Systems aufweist, **dadurch gekennzeichnet, dass** sie enthält:
a) mindestens ein amphiphiles Polymer der Acrylamido-2-methylpropansulfonsäure, das ganz oder teilweise neutralisiert, vernetzt oder nicht vernetzt ist, wobei das Polymer einen hydrophilen Bereich und gleichzeitig einen hydrophoben Bereich aufweist, der mindestens eine Kohlenwasserstoffkette mit mindestens 7 Kohlenstoffatomen umfasst; und
b) mindestens ein wasserlösliches Silicon, das mindestens eine endständige oder als Seitenkette vorliegende, einwertige Polyoxyalkylengruppe aufweist.

2. Zusammensetzung nach Anspruch 1, **dadurch gekennzeichnet, dass** das AMPS-Polymer ganz oder teilweise mit einer anorganischen oder organischen Base neutralisiert ist.

3. Zusammensetzung nach Anspruch 2, **dadurch gekennzeichnet, dass** die anorganische Base unter Natriumhydroxid, Kaliumhydroxid oder Ammoniak ausgewählt ist.

4. Zusammensetzung nach Anspruch 2, **dadurch gekennzeichnet, dass** die organische Base unter Mono-, Di- oder Triethanolamin, Aminomethylpropandiol, N-Methylglucamin, basischen Aminosäuren und ihren Gemischen ausgewählt ist.

5. Zusammensetzung nach einem der Ansprüche 1 bis 4, wobei das AMPS-Polymer zu mindestens 90 % neutralisiert ist.

6. Zusammensetzung nach einem der Ansprüche 1 bis 5, wobei das AMPS-Polymer vernetzt ist und das Vernetzungsmittel unter den Verbindungen mit mehreren olefinisch ungesättigten Bindungen ausgewählt ist, die häufig zur Vernetzung der durch radikalische Polymerisation hergestellten Polymere verwendet werden.

7. Zusammensetzung nach Anspruch 6, wobei das Vernetzungsmittel unter Divinylbenzol, Diallylether, Dipropylenglycoldiallylether, Polyglycoldiallylethern, Triethylenglycoldivinylether, Hydrochinondiallylether, Ethylenglycoldi(meth)acrylat oder Tetraethylen-glycoldi(meth)acrylat, Trimethylolpropantriacrylat, Methylen-bis-acrylamid, Methylen-bis-methacrylamid, Triallylamin, Triallylcyanurat, Diallylmaleat, Tetraallylethylendiamin, Tetraallyloxyethan, Trimethylolpropandiallylether, Allyl-(meth)acrylat, Allylethern von Alkoholen der Zuckergruppe, oder anderen Allyl- oder Vinylethern von mehrwertigen Alkoholen, sowie Allylestern der Derivate der Phosphorsäure und/oder der Vinylphosphonsäure, oder deren Gemischen ausgewählt ist.

8. Zusammensetzung nach Anspruch 6, wobei das Vernetzungsmittel unter Methylen-bis-acrylamid, Allylmethacrylat oder Trimethylolpropantriacrylat (TMPTA) ausgewählt ist.

9. Zusammensetzung nach einem der Ansprüche 7 bis 8, wobei der Vernetzungsgrad, bezogen auf das Polymer, im Allgemeinen im Bereich von 0,01 bis 10 Mol-% und insbesondere im Bereich von 0,2 bis 2 Mol-% liegt.

10. Zusammensetzung nach einem der Ansprüche 1 bis 9, wobei das amphiphile Polymer von AMPS mindestens eine Fettkette umfasst, die 7 bis 30 Kohlenstoffatome, vorzugsweise 7 bis 22 Kohlenstoffatome und noch bevorzugter 7 bis 18 Kohlenstoffatome und insbesondere 12 bis 18 Kohlenstoffatome enthält.

11. Zusammensetzung nach einem der Ansprüche 1 bis 10, wobei das amphiphile Polymer von AMPS ein Molekulargewicht von 50 000 bis 10 000 000, bevorzugter von 100 000 bis 8 000 000 und noch bevorzugter von 100 000 bis 7 000 000 aufweist.

12. Zusammensetzung nach einem der Ansprüche 1 bis 11, wobei die amphiphilen Polymere von AMPS unter den statistischen amphiphilen Polymeren von AMPS ausgewählt sind, die durch Reaktion mit einem n-Monoalkylamin oder Di-n-alkylamin mit 6 bis 22 Kohlenstoffatomen modifiziert sind und ein oder mehrere hydrophile Monomere mit ethylenisch ungesättigter Bindung enthalten können.

13. Zusammensetzung nach einem der Ansprüche 1 bis 12, wobei die amphiphilen Polymere von AMPS unter den Polymeren von AMPS und mindestens einem Monomer mit ethylenisch ungesättigter Bindung, das mindestens einen hydrophoben Teil mit 7 bis 30 Kohlenstoffatomen und bevorzugter 7 bis 22 Kohlenstoffatomen und noch bevorzugter 7 bis 18 Kohlenstoffatomen und insbesondere 12 bis 18 Kohlenstoffatomen enthält, und gegebenenfalls einem oder mehreren hydrophilen Comonomeren mit ethylenisch ungesättigter Bindung ausgewählt sind.

14. Zusammensetzung nach Anspruch 13, wobei die Monomere mit ethylenisch ungesättigter Bindung, die mindestens einen hydrophoben Teil mit 7 bis 30 Kohlenstoffatomen umfassen, unter den Acrylaten oder Acrylamiden der folgenden Formel (1) ausgewählt sind: 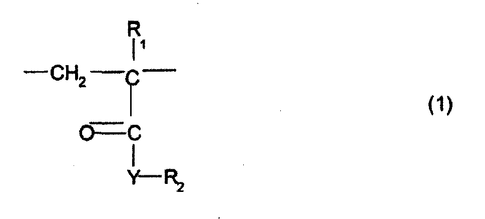 worin R₁ ein Wasserstoffatom, eine geradkettige oder verzweigte C₁₋₆-Alkylgruppe (vorzugsweise Methyl) bedeutet; Y O oder NH ist; und R₂ eine hydrophobe Gruppe bedeutet, die eine Fettkette mit 7 bis 22 Kohlenstoffatomen, vorzugsweise 7 bis 18 Kohlenstoffatomen und insbesondere 12 bis 18 Kohlenstoffatomen umfasst.

15. Zusammensetzung nach Anspruch 20, wobei die hydrophobe Gruppe R₂ unter den geradkettigen oder verzweigten, gesättigten oder ungesättigten C₇₋₁₈-Alkylgruppen; den C₇₋₁₈-alkyl-perfluorierten Gruppen; der Cholesterylgruppe oder einem Cholesterinester; oder aromatischen polycyclischen Gruppen ausgewählt ist.

16. Zusammensetzung nach Anspruch 14 oder 15, wobei die hydrophobe Gruppe R₂ darüber hinaus mindestens eine Alkylenoxideinheit und vorzugsweise eine polyalkoxylierte Kette umfasst.

17. Zusammensetzung nach Anspruch 16, wobei die Anzahl der alkoxylierten Einheiten im Bereich von 1 bis 30 Mol und vorzugsweise 1 bis 25 Mol und noch bevorzugter 3 bis 20 Mol liegt.

18. Zusammensetzung nach einem der Ansprüche 14 bis 17, wobei die amphiphilen Polymere von AMPS amphiphile Copolymere sind, bestehend aus:
(a) Einheiten der 2-Acrylamido-2-methylpropansulfonsäure (AMPS) der folgenden Formel (2): 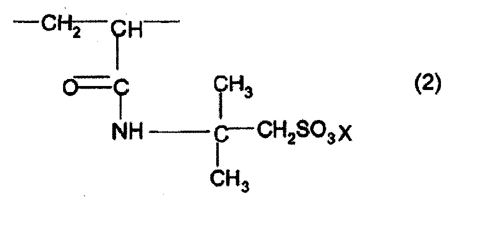 in der X⁺ ein Proton, ein Alkalimetallkation, ein Erdalkalimetallkation oder ein Ammoniumion ist;
(b) Einheiten der folgenden Formel (3): 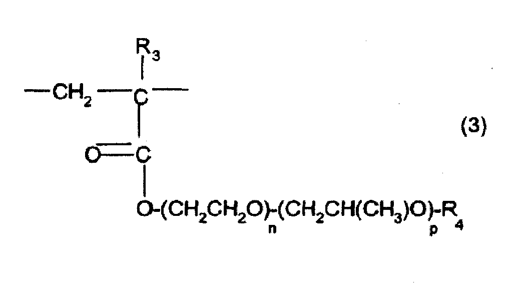 in der n und p jeweils unabhängig voneinander die Molzahl angeben und im Bereich von 0 bis 30, vorzugsweise von 1 bis 25 und noch bevorzugter von 3 bis 20 liegen, mit der Maßgabe, dass n + p kleiner oder gleich 30, vorzugsweise kleiner als 25 und noch besser kleiner als 20 ist; R₃ ein Wasserstoffatom, eine geradkettige oder verzweigte C₁₋₆-Alkylgruppe (vorzugsweise Methyl) bedeutet; und R₄ eine geradkettige oder verzweigte Alkylgruppe bedeutet, die 7 bis 22 Kohlenstoffatome, vorzugsweise 7 bis 18 Kohlenstoffatome und noch besser 12 bis 18 Kohlenstoffatome umfasst.

19. Zusammensetzung nach Anspruch 18, wobei X⁺ Natrium oder Ammonium ist.

20. Zusammensetzung nach Anspruch 17 oder 18, wobei die Einheit der Formel (3) ausgewählt ist unter:
· Estern von (Meth)acrylsäure und einem Cio-₁₈-Fettalkohol, die mit 8 EO polyethoxyliert sind;
· Estern von (Meth)acrylsäure und einem mit 8 EO polyethoxylierten C₁₁-Oxoalkohol;
· Estern von (Meth)acrylsäure und einem mit 7 EO polyethoxylierten C₁₂₋₁₄-Fettalkohol;
· Estern von (Meth)acrylsäure und einem mit 11 EO polyethoxylierten C₁₂₋₁₄-Fettalkohol;
· Estern von (Meth)acrylsäure und einem mit 8 EO polyethoxylierten C₁₆₋₁₈-Fettalkohol;
· Estern von (Meth)acrylsäure und einem mit 15 EO polyethoxylierten C₁₆₋₁₈-Fettalkohol;
· Estern von (Meth)acrylsäure und einem mit 11 EO polyethoxylierten C₁₆₋₁₈-Fettalkohol;
· Estern von (Meth)acrylsäure und einem mit 20 EO polyethoxylierten C₁₆₋₁₈-Fettalkohol;
· Estern von (Meth)acrylsäure und einem mit 25 EO polyethoxylierten C₁₆₋₁₈-Fettalkohol;
· Estern von (Meth)acrylsäure und einem mit 25 EO polyethoxylierten C₁₈₋₂₂-Fettalkohol und/oder einem mit 25 EO polyethoxylierten C₁₆₋₁₈-Isoalkohol.

21. Zusammensetzung nach einem der Ansprüche 14 bis 20, wobei die amphiphilen Polymere von AMPS ausgewählt sind unter:
(i) den nicht vernetzten Polymeren, in denen p = 0, n = 7 oder 25, R₃ Methyl und R₄ ein Gemisch aus C₁₂₋₁₄-Alkyl oder C₁₆₋₁₈-Alkyl bedeutet,
(ii) den vernetzten Polymeren, in denen p = 0, n = 8 oder 25, R₃ Methyl und R₄ ein Gemisch aus C₁₆₋₁₈-Alkyl bedeutet.

22. Zusammensetzung nach einem der Ansprüche 18 bis 21, wobei der molare Anteil der Einheiten der Formel (3) im Bereich von 0,1 bis 50 %, genauer von 1 bis 25 % und insbesondere von 3 bis 10 % liegt.

23. Zusammensetzung nach einem der Ansprüche 18 bis 21, wobei der molare Anteil der Einheiten der Formel (3) im Bereich von 50,1 bis 99,9 % und besonders von 60 bis 95 % und noch bevorzugter von 65 bis 90 % liegt.

24. Zusammensetzung nach einem der Ansprüche 1 bis 23, wobei die Polymere der AMPS in einer auf die wirksame Substanz bezogenen Menge von 0,01 bis 20 Gew.-%, bevorzugter von 0,1 bis 10 Gew.-%, noch bevorzugter von 0,1 bis 5 Gew.-% und besonders bevorzugt von 0,5 bis 2 Gew.-%, bezogen auf das Gesamtgewicht der Zusammensetzung, enthalten sind.

25. Zusammensetzung nach einem der Ansprüche 1 bis 24, wobei das wasserlösliche Silicon, das mindestens eine endständige oder als Seitenkette vorliegende, einwertige Polyoxyalkylengruppe aufweist, unter den Verbindungen der folgenden allgemeinen Formeln (I), (II) ausgewählt ist: 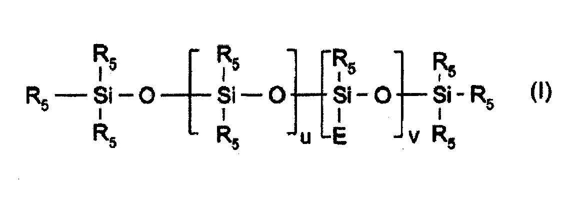 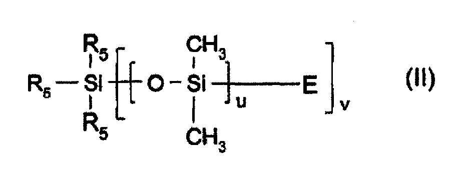 in den Formeln:
· die Gruppen R₅, die gleich oder verschieden sind, bedeuten eine einwertige Kohlenwasserstoffgruppe, die unter den Alkylgruppen, Arylgruppen und Aralkylgruppen ausgewählt sind, die nicht mehr als 10 Kohlenstoffatome aufweisen und vorzugsweise unter den C₁₋₄-Alkylgruppen ausgewählt sind; wobei einige Gruppen R₅ ferner eine Ethylcyclohexylmonoxid enthalten können und in einem geringen Anteil in der Polysiloxankette enthalten sind;
· u liegt im Bereich von 10 bis 150, vorzugsweise 25 bis 100 und noch bevorzugter 65 bis 85;
· v liegt im Bereich von 3 bis 12, vorzugsweise 4 bis 10 und noch bevorzugter 5 bis 8;
· E bedeutet eine Gruppe -CₓH₂ₓ-(OC₂H₄)y-(OC₃H₆)_{z}-OR₆ mit
· x liegt im Bereich von 1 bis 8;
· y > 0 und z ≥ O; wobei y und z so ausgewählt sind, dass die Molmasse der Gruppe E im Bereich von 200 bis 10000 liegt;
· R₆ bedeutet Wasserstoff; eine geradkettige oder verzweigte C₁₋₈-Alkylgruppe, eine geradkettige oder verzweigte C₂₋₈-Acylgruppe.

26. Zusammensetzung nach Anspruch 27, wobei die Gruppen R₅ eine niedere C₁₋₄-Alkylgruppe bedeutet; R₆ Wasserstoff, eine C₁₋₄-Alkylgruppe oder eine C₂₋₄-Acylgruppe bedeutet; x im Bereich von 2 bis 4 liegt und y und z so ausgewählt sind, dass die Molmasse der Gruppe E im Bereich von 350 bis 4000 liegt.

27. Zusammensetzung nach Anspruch 26, wobei alle Gruppen R₅ Methyl bedeuten; R₆ Wasserstoff, Methyl oder Acetyl bedeutet; und x 3 ist.

28. Zusammensetzung nach einem der Ansprüche 1 bis 27, wobei das wasserlösliche Silicon, das mindestens eine endständige oder als Seitenkette vorliegende, einwertige Polyoxyalkylengruppe aufweist, in Konzentrationen von 0,01 bis 20 Gew.-%, vorzugsweise 0,1 bis 15 Gew.-% und insbesondere 0,5 bis 5 Gew.-%. bezogen auf das Gesamtgewicht der Zusammensetzung, enthalten ist.

29. Zusammensetzung nach einem der Ansprüche 1 bis 28, **dadurch gekennzeichnet, dass** das UV-Filtersystem mindestens einen zusätzlichen, organischen oder anorganischen Sonnenschutzfilter umfasst, der im UV-A- und/oder UV-B-Bereich wirksam und wasserlöslich, fettlöslich oder in den üblicherweise verwendeten kosmetischen Lösungsmitteln unlöslich ist.

30. Zusammensetzung nach Anspruch 29, wobei die ergänzenden organischen Filter unter den Anthranilaten; Zimtsäurederivaten; Dibenzoylmethanderivaten; Salicylsäurederivaten, Campherderivaten; Triazinderivaten; Benzophenonderivaten; Derivaten von β,β-Diphenylacrylat; Benzotriazolderivaten; Benzalmalonatderivaten; Derivaten von Benzimidazol; Imidazolinen; Bis-benzoazolylderivaten; Derivaten von p-Aminobenzoesäure (PABA); Benzoxazolderivaten; Derivaten von Methylen-bis(hydroxyphenyl-benzotriazol); Polymerfiltern und Siliconfiltern; dimeren Derivaten von α-Alkylstyrol; 4,4-Diarylbutadienen und ihren Gemischen ausgewählt sind.

31. Zusammensetzung nach Anspruch 30, wobei die ergänzenden organischen Filter ausgewählt sind unter:
Ethylhexyl Salicylate,
Ethylhexyl Methoxycinnamate,
Octocrylene,
Butyl Methoxydibenzoylmethane,
Phenylbenzimidazole Sulfonic Acid,
Benzophenone-3,
Benzophenone-4,
Benzophenone-5,
n-Hexyl-2-(4-diethylamino-2-hydroxybenzoyl)-benzoat,
4-Methylbenzylidene Camphor,
Terephthalylidene Dicamphor Sulfonic Acid,
Disodium Phenyl Dibenzimidazole Tetrasulfonate,
2,4,6-Tris-(diisobutyl-4'amino-benzalmalonat)-s-triazin,
Anisotriazine,
Ethylhexyl Triazone,
Diethylhexyl Butamido Triazone,
Methylen bis-Benzotriazolyl Tetramethylbutylphenol,
Drometrizole Trisiloxane,
Polysilicone-15,
1,1-Dicarboxy-(2,2'-dimethyl-propyl)-4,4-diphenylbutadien,
2,4-Bis[5-1-(dimethylpropyl)benzoxazol-2-yl-(4-phenyl)-imino]-6-(2-ethylhexyl)-imino-1,3,5-triazin und ihren Gemischen.

32. Zusammensetzung nach Anspruch 29, wobei die anorganischen Filter unter den Pigmenten oder Nanopigmenten von Metalloxiden, die umhüllt oder nicht umhüllt sind, ausgewählt sind.

33. Zusammensetzung nach Anspruch 32, wobei die ergänzenden anorganischen Filter Nanopigmente von amorphem oder kristallinem Titanoxid in Form von Rutil und/oder Anatas, Eisenoxid, Zinkoxid, Zirconiumoxid oder Ceroxid sind.

34. Zusammensetzung nach einem der Ansprüche 1 bis 33, **dadurch gekennzeichnet, dass** sie darüber hinaus mindestens ein Bräunungsmittel und/oder Mittel zur künstlichen Braunfärbung der Haut enthält.

35. Zusammensetzung nach einem der Ansprüche 1 bis 34, **dadurch gekennzeichnet, dass** sie darüber hinaus mindestens einen kosmetischen Zusatzstoff enthält, der unter den organischen Lösungsmitteln, ionischen oder nichtionischen Verdickungsmitteln, reizlindernden Stoffen, Feuchthaltemitteln, Trübungsmitteln, Stabilisatoren, Emollientien, Siliconen, insektenabwehrenden Wirkstoffen, Parfums, Konservierungsmitteln, grenzflächenaktiven Stoffen, Füllstoffen, Wirkstoffen, Pigmenten, Polymeren, Treibmitteln, Alkalisierungsmitteln oder Ansäuerungsmitteln oder beliebigen anderen Bestandteilen, die gewöhnlich auf dem Gebiet der Kosmetik und/oder Dermatologie verwendet werden, ausgewählt ist.

36. Zusammensetzung nach einem der Ansprüche 1 bis 35, **dadurch gekennzeichnet, dass** sie als Lotion oder Serum, als wässriges Gel, in Form einer Öl-in-Wasser-Emulsion oder Wasser-in-Öl-Emulsion; als multiple Emulsion, als Mikroemulsion; in der Form von Vesikeldispersionen vom ionischen und/ oder nichtionischen Typ oder in Form von Wachs/wässrige Phase-Dispersionen vorliegt.

37. Zusammensetzung nach einem der Ansprüche 1 bis 36, **dadurch gekennzeichnet, dass** sie als Öl-in-Wasser-Emulsion oder Wasser-in-Öl-Emulsion vorliegt, die höchstens 1 Gew.-% eines emulgierenden grenzflächenaktiven Stoffes, bezogen auf das Gesamtgewicht der Zusammensetzung, enthält.

38. Verwendung einer Zusammensetzung, wie sie in einem der Ansprüche 1 bis 37 definiert wurde, zur Herstellung von Produkten für die kosmetische Behandlung der Haut, der Lippen und der Haare, wobei die Kopfhaut eingeschlossen ist, insbesondere zum Schutz und/oder zur Pflege der Haut, der Lippen und/oder der Haare und/oder zum Schminken der Haut und/oder der Lippen.

39. Verwendung der Kombination aus mindestens einem amphiphilen Polymer der Acrylamido-2-methyl-propansulfonsäure mit mindestens einer Fettkette, das ganz oder teilweise neutralisiert, vernetzt oder unvernetzt ist, und mindestens einem wasserlöslichen Silicon, das mindestens eine endständige oder als Seitenkette vorliegende, einwertige Polyoxyalkylengruppe aufweist, wie sie in einem der vorhergehenden Ansprüche definiert wurden, in einer wässrigen kosmetischen Zusammensetzung, die, bezogen auf das Gesamtgewicht der Zusammensetzung, 0,1 bis 30 Gew.-% eines Lichtschutzsystems aufweist, das befähigt ist, die UV-Strahlung zu filtern, um den Lichtschutzfaktor zu erhöhen.
